# EUROPEAN PATENT APPLICATION

(11) **EP 4 563 171 A1**
(43) Date of publication of application: **04.06.2025**
(21) Application number: 24215506.7
(22) Date of filing: 26.11.2024
(51) Int. Cl.: A61M 5/142, A61M 5/14, A61M 39/08

(54) **FLUID PUMP WITH INTEGRAL TUBING RETENTION DEVICE AND RELATED METHODS**

(30) Priority: 28.11.2023 US 202318521718
(71) Applicant: Laborie Medical Technologies Corp., Portsmouth, NH 03801 (US)
(72) Inventor: KOEHN, Joanna Rosenbaum, Beloit, WI 53511 (US); BOMGAARS, Grant, Kildeer, IL 60047 (US)
(74) Representative: Reddie & Grose LLP

(57) **Abstract**

A tubing retention device for retaining a T-shaped tubing assembly includes a base member having a first tubing recess, a second tubing recess oriented perpendicular to the first tubing recess, and first and second relief shelves disposed at opposite ends of the first tubing recess and recessed relative to the bottom surface of the first tubing recess. A movable locking element is configured to move between an unlocked first position and a locked second position and is configured to lock a T-shaped tubing assembly in the tubing retention device in the locked second position. A fluid pump includes a housing and such a tubing retention device mounted to the housing. A T-shaped tubing assembly may be secured within such a retention device by positioning the T-shaped tubing assembly in the first and second tubing recesses, and moving the locking element from the unlocked first position to the locked second position.

## Description

### PRIORITY CLAIM

This application claims the benefit of the filing date of United States Patent Application Serial No. 18/521,718, filed November 28, 2023, for "FLUID PUMP WITH INTEGRAL TUBING RETENTION DEVICE AND RELATED METHODS," the disclosure of which is hereby incorporated herein in its entirety by this reference.

### TECHNICAL FIELD

This disclosure relates generally to fluid pumps and to tubing retention devices used in conjunction or forming a part of such fluid pumps.

### BACKGROUND

Fluid pumps are used in various industrial applications, such as in the medical industry. In many applications, tubing assemblies, which may be single-use disposable tubing assemblies are used in conjunction with the fluid pump. Supporting the tubing assemblies during use of the fluid pump in a manner that secures the tubing in a safe, clean, reliable, and convenient manner is important, especially when the fluid being handled contains potentially hazardous biomedical waste, and when the attention of the user of the fluid pump must be on the medical procedure being performed and not on the fluid pump or accessories thereof.

### DISCLOSURE

In some embodiments, the present disclosure includes a tubing retention device for retaining a T-shaped tubing assembly. The tubing retention device includes a base member having external surfaces defining a first tubing recess, a second tubing recess oriented at least substantially perpendicular to the first tubing recess, a first relief shelf disposed at a first end of the first tubing recess and recessed relative to a bottom surface of the first tubing recess, and a second relief shelf disposed at a second end of the first tubing recess and recessed relative to the bottom surface of the first tubing recess. The tubing retention device further includes a movable locking element configured to move between an unlocked first position and a locked second position. The movable locking element is configured to lock a T-shaped tubing assembly in the tubing retention device when the T-shaped tubing assembly is positioned within the first tubing recess and the second tubing recess and the movable locking element is moved to the locked second position.

In additional embodiments, the present disclosure includes a fluid pump including a housing, and such a tubing retention device mounted to the housing.

In yet further embodiments, the present disclosure includes a method of securing a T-shaped tubing assembly within a retention device. In accordance with the method, a T-shaped tubing assembly is positioned within a first tubing recess and a second tubing recess in a base member. The second tubing recess is oriented at least substantially perpendicular to the first tubing recess. The base member has external surfaces defining a first relief shelf disposed at a first end of the first tubing recess and recessed relative to a bottom surface of the first tubing recess, and a second relief shelf disposed at a second end of the first tubing recess and recessed relative to the bottom surface of the first tubing recess. A movable locking element carried by the base member is then moved from an unlocked first position to a locked second position. The movable locking element locks the T-shaped tubing assembly in the retention device while the movable locking element is in the locked second position.

### BRIEF DESCRIPTION OF THE DRAWINGS

For a detailed understanding of the present disclosure, reference should be made to the following detailed description, taken in conjunction with the accompanying drawings, in which like elements have generally been designated with like numerals, and wherein:
FIG. 1 is a perspective view of a fluid pump having a tubing retention device in accordance with embodiments of the present disclosure;
FIG. 2 is a perspective view of the tubing retention device of the pump of FIG. 1 shown in isolation;
FIG. 3 is a top view of the tubing retention device;
FIGS. 4 and 5 are left and right side views, respectively, of the tubing retention device;
FIG. 6 is a cross-sectional side view of the tubing retention device;
FIG. 7 is a bottom view of the tubing retention device;
FIG. 8 is a front side view of the movable locking element of the tubing retention device;
FIG. 9 is a right side view of the movable locking element of the tubing retention device;
FIG. 10 is an exploded view of the tubing retention device;
FIG. 11 is a front view of the tubing retention device;
FIG. 12 is a cross-sectional side view of the tubing retention device taken along the plane A-A in FIG. 11;
FIG. 13 is a perspective view of the tubing retention device with a T-shaped tubing assembly retained therein;
FIG. 14 is a top view of the tubing retention device with a T-shaped tubing assembly retained therein; and
FIG. 15 illustrates a known T-shaped tubing assembly that may be retained in a tubing retention device in accordance with embodiments of the present disclosure.

### MODE(S) FOR CARRYING OUT THE INVENTION

The illustrations presented herein are not actual views of any fluid pump or tubing retention device, or any component thereof, but are merely idealized representations, which are employed to describe embodiments of the present invention.

As used herein, the singular forms following "a," "an," and "the" are intended to include the plural forms as well, unless the context clearly indicates otherwise.

As used herein, the term "may" with respect to a material, structure, feature, or method act indicates that such is contemplated for use in implementation of an embodiment of the disclosure, and such term is used in preference to the more restrictive term "is" so as to avoid any implication that other compatible materials, structures, features, and methods usable in combination therewith should or must be excluded.

As used herein, any relational term, such as "first," "second," "top," "bottom," "upper," "lower," "above," "beneath," "side," "upward," "downward," *etc.,* is used for clarity and convenience in understanding the disclosure and accompanying drawings, and does not connote or depend on any specific preference or order, except where the context clearly indicates otherwise. For example, these terms may refer to an orientation of elements of any fluid pump or tubing retention device when utilized in a conventional manner. Furthermore, these terms may refer to an orientation of elements of any fluid pump or tubing retention device as illustrated in the drawings.

As used herein, the term "substantially" in reference to a given parameter, property, or condition means and includes to a degree that one skilled in the art would understand that the given parameter, property, or condition is met with a small degree of variance, such as within acceptable manufacturing tolerances. By way of example, depending on the particular parameter, property, or condition that is substantially met, the parameter, property, or condition may be at least 90.0% met, at least 95.0% met, at least 99.0% met, or even at least 99.9% met.

As used herein, the term "about" used in reference to a given parameter is inclusive of the stated value and has the meaning dictated by the context (*e.g*., it includes the degree of error associated with measurement of the given parameter, as well as variations resulting from manufacturing tolerances, *etc*.).

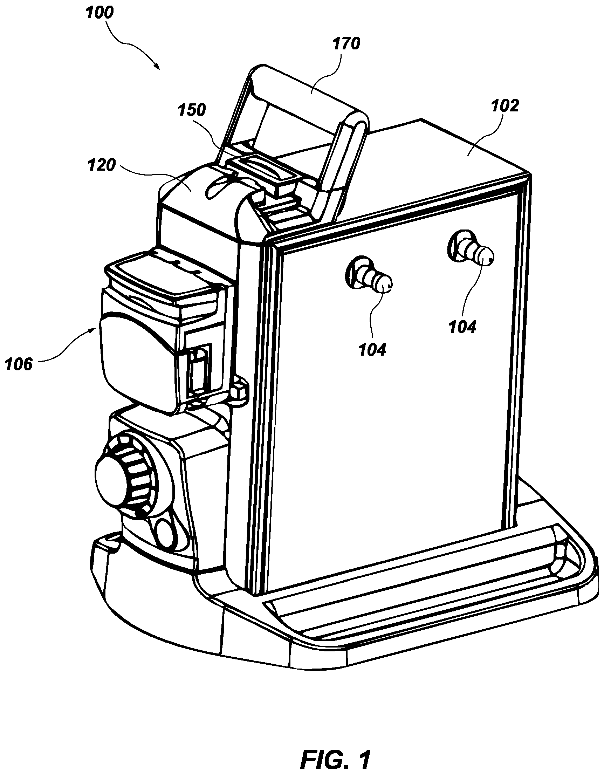

FIG. 1 is a perspective view of a fluid pump 100 that includes a housing 102 and a tubing retention device 120 mounted to the housing 102. The tubing retention device 120 is configured to hold and retain a T-shaped tubing assembly 200 that is used in conjunction with the fluid pump 100. The fluid pump 100 shown in FIG. 1 is a fluid pump used in the medical industry. In particular, the fluid pump 100 described herein is a peristaltic fluid pump used in centesis procedures (*e.g*., paracentesis, thoracentesis, *etc*.) to remove bodily fluid from the body of a patient.

Peristaltic pumps are also known in industry as hose pumps, tube pumps, or roller pumps. Peristaltic pumps operate by compressing a section of tubing successively by two or more pressing "shoes," which are typically mounted on a rotating wheel. The first shoe will create a vacuum and attract the pumped fluid into the tube by compressing the tubing and rolling relative to the tubing such that the compressed section of tubing is advanced along the length of the tubing. Once the pumped fluid has entered the tube, the roller(s) pass along and compress the tube in successive fashion. This rolling compressive motion on the tube forces fluid flow from an inlet of the tube toward an outlet of the tube.

The fluid pump 100 of FIG. 1 is configured for use with two disposable fluid containers. In particular, the housing 102 includes a pair of posts 104 on each opposing lateral side of the housing 102, which are located and configured to cooperate with features of a disposable bag (not shown). In particular, a disposable bag may be suspended from each pair of posts, such that two disposable bags are carried by the housing 102 during operation of the pump. The T-shaped tubing assembly 200 has an inlet tubing section 202 and two outlet tubing sections 204. During use of the fluid pump 100, the distal end of the inlet tubing section 202 is coupled to a device that is inserted into the body of a patient to access the bodily fluid to be removed. The distal end of each of the outlet tubing sections 204 is connected with one of the disposable bags carried on the housing 102 of the fluid pump 100. A portion of the inlet tubing section 202 proximate the fluid pump 100 is positioned within a peristaltic pumping mechanism 106 of the fluid pump 100. During operation, the peristaltic pumping mechanism 106 pumps the fluid from the body of the patient through the inlet tubing section and into one or both of the disposable bags carried on the housing 102 of the fluid pump 100. After the procedure, the bags containing fluid and the T-shaped tubing assembly 200 are discarded in accordance with standard procedures for disposing of medical waste. A new sterilized T-shaped tubing assembly 200 and one or more disposable bags are used for each medical procedure.

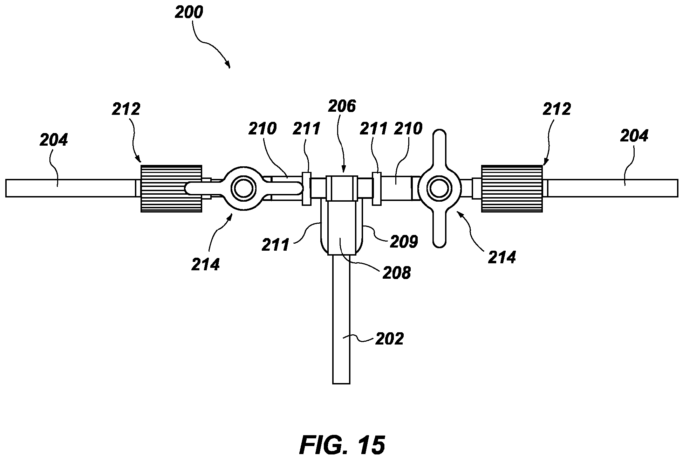

Referring briefly to FIG. 15, the T-shaped tubing assembly 200 includes a rigid plastic T-shaped manifold 206. The inlet tubing section 202 may be permanently attached to the vertical (from the perspective of FIG. 15) branch 208 of the T-shaped manifold 206. Luer connectors 212 are provided on the ends of the horizontal (from the perspective of FIG. 15) branches 210 of the T-shaped manifold 206. In addition, valves 214 are provided on each of the horizontal branches 210 of the T-shaped manifold 206 between the vertical branch 208 of the manifold 206 and the luer connectors 212, such that the fluid being pumped from the inlet tubing section 202 can be diverted to either of the disposable bags or to both of the disposable bags during operation of the fluid pump 100. Typically, during normal operation, only one disposable bag would be filled at a time. Once that bag is full, the fluid flow may be switched to the other disposable bag, and the full disposable bag may be removed and replaced while the other disposable bag continues to be filled.

Although the fluid pump 100 described with reference to FIG. 1 is a peristaltic fluid pump for use in medical procedures, the tubing retention device 120 may be employed with any other type of fluid pump in industry that is used in conjunction with a T-shaped tubing assembly like that shown in FIG. 15, and any such fluid pump having a tubing retention device 120 as described herein are withing scope of the present disclosure.

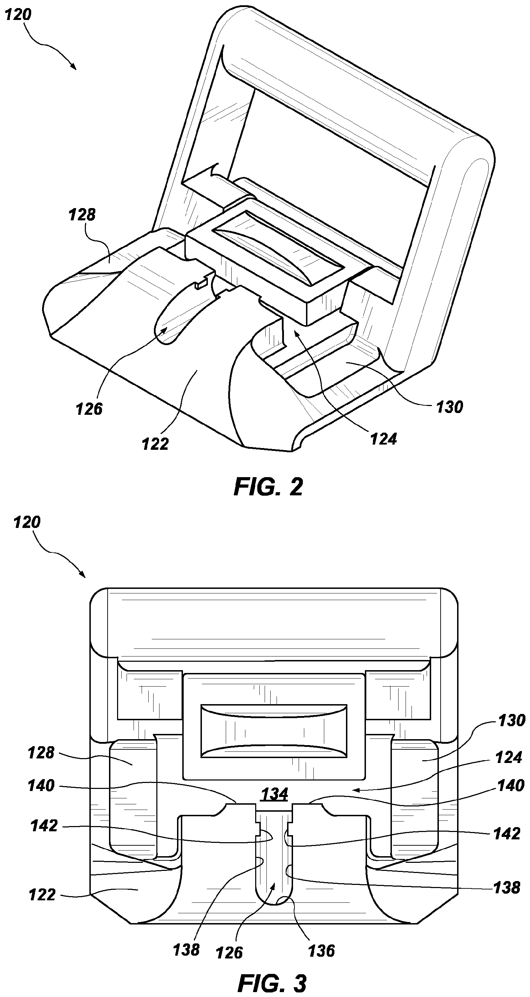

FIG. 2 is a perspective view and FIG. 3 is a top view of the tubing retention device 120 of the fluid pump 100 of FIG. 1, the tubing retention device 120 being shown in isolation. As shown in FIGS. 2 and 3, the tubing retention device 120 includes a base member 122 having external surfaces defining a first tubing recess 124 and a second tubing recess 126 oriented at least substantially perpendicular to the first tubing recess 124.

The tubing retention device 120 further includes a movable locking element 150 configured to move between an unlocked first position and a locked second position, as is discussed in further detail below. The movable locking element 150 is configured to lock a T-shaped tubing assembly 200 in the tubing retention device 120 when the T-shaped tubing assembly 200 is positioned within the first tubing recess 124 and the second tubing recess 126 and the movable locking element 150 is moved to the locked second position.

Optionally, a handle 170 may extend from the tubing retention device 120 to facilitate movement of the fluid pump 100.

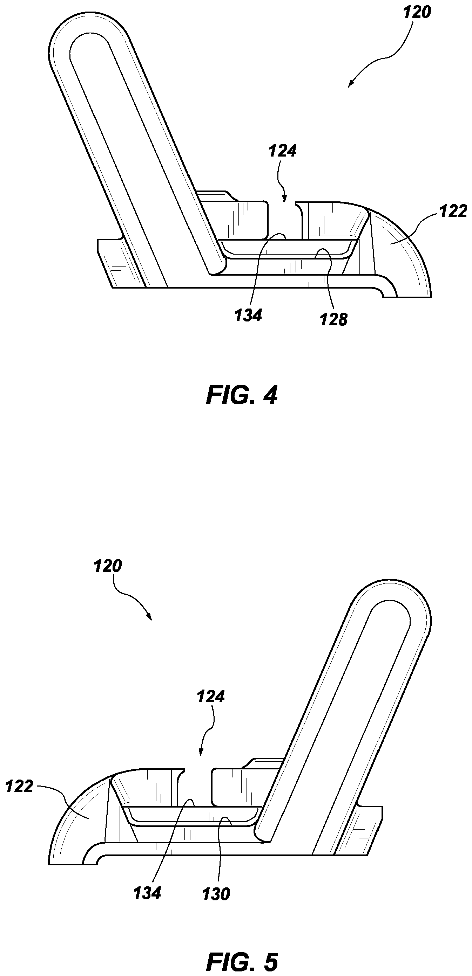

The external surfaces of the base member 122 define a first relief shelf 128 and second relief shelf 130. The first relief shelf 128 is disposed at a first end of the first tubing recess 124, and is recessed relative to a bottom surface 134 of the first tubing recess 124. Similarly, the second relief shelf 130 is disposed at an opposite second end of the first tubing recess 124, and is also recessed relative to the bottom surface 134 of the first tubing recess 124. For example, the surfaces of the base member 122 defining the first relief shelf 128 and the second relief shelf 130 may be recessed relative to the surface of the base member 122 defining the bottom surface 134 within the first tubing recess by a distance D in a range extending from about 6.5 mm to about 12.5 mm (e.g., about 9.5 mm), as shown in the side views of FIGS. 4 and 5.

As can be seen in FIGS. 2-5, each of the first relief shelf 128, the second relief shelf 130, and the bottom surface 134 within the first tubing recess 124 may be at least substantially planar, and they may be oriented parallel to one another. The first relief shelf 128 and the second relief shelf 130 may be coplanar and recessed relative to the bottom surface 134 of the first tubing recess 124 by the same distance D. A surface of the base member 122 defining the second tubing recess 126 is semicylindrical at a bottom 136 of the second tubing recess 126 and planar at opposing sides 138 of the second tubing recess 126, as is best seen in FIGS. 2 and 3.

Optionally, the base member 122 may further include at least one first tubing retention protrusion 140 protruding over at least a portion of the first tubing recess 124, and at least one second tubing retention protrusion 142 protruding over at least a portion of the second tubing recess 126. The first and second retention protrusions 140, 142 are configured to hinder removal of a T-shaped tubing assembly 200 from within the first tubing recess 124 and the second tubing recess 126 while the movable locking element 150 is in the locked second position. In the embodiment shown in the figures, the base member includes two first tubing retention protrusions 140 protruding over the first tubing recess 124 on opposite sides of the second tubing recess 126, respectively, and two second tubing retention protrusions 142 each extending over the second tubing recess 126 on opposite sides of the second tubing recess 126. In other embodiments, more or less tubing retention protrusions may be employed.

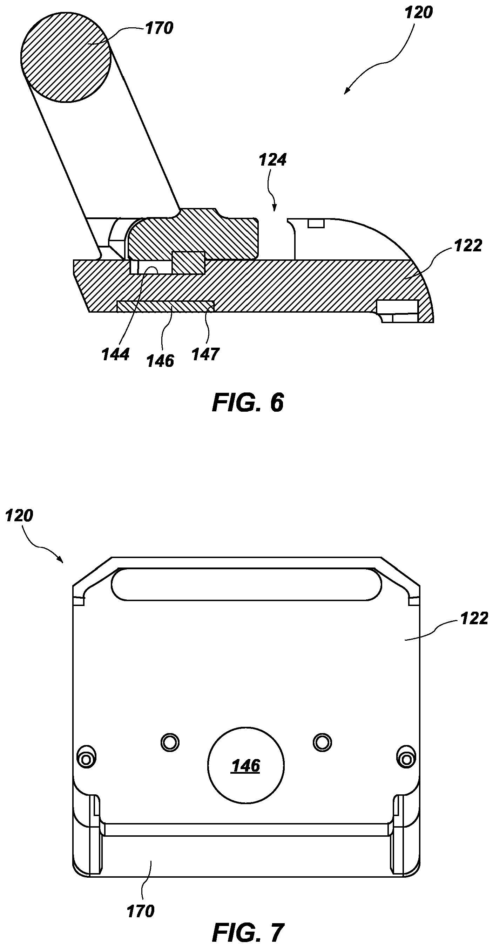

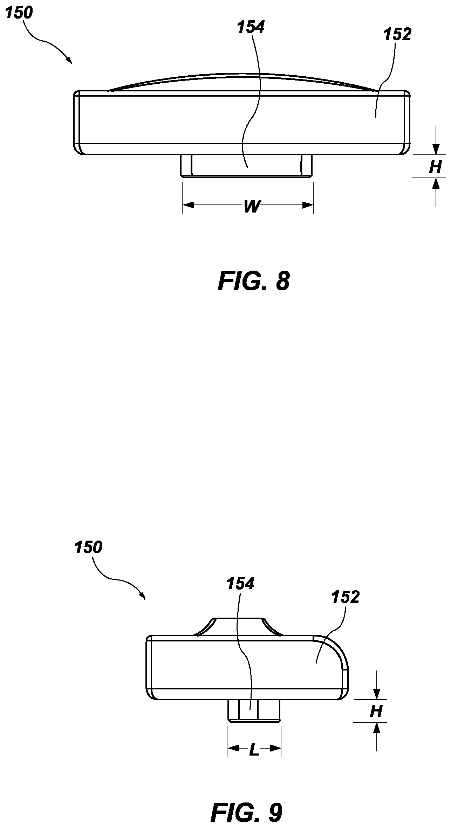

FIG. 6 is a cross-sectional side view of the tubing retention device 120. As shown therein, the movable locking element 150 includes a main body 152, and a protrusion 154 extending downward from the bottom surface of the main body 152. A recess 144 is provided in an upper surface of the base member 122, which is configured to receive the protrusion 154 therein. The recess 144 and the protrusion 154 are configured to have complementary sizes and shapes. In particular, as shown in FIG. 8 and 9, the protrusion 154 has a height H, a width W (FIG. 8), and a length L (FIG. 9). The recess 144 has a very similar height and width but has a length that is longer than the length L of the protrusion 154 as shown in FIG. 6. In this configuration, the protrusion 154 and the recess 144 are configured in a tongue-and-groove type manner that allows the movable locking element 150 to slide relative to the base member 122 in the left and right directions (from the perspective of FIG. 6), but not in other directions in the horizontal plane of FIG. 6. The first unlocked position of the movable locking element 150 is the leftmost position of the movable locking element 150 from the perspective of FIG. 6, and the second locked position of the movable locking element 150 is the rightmost position of the movable locking element 150 from the perspective of FIG. 6. The moveable locking element 150 is shown in the second locked position in each of FIGS. 2-6.

The base member 122 may further comprise a biasing mechanism configured to bias the movable locking element 150 in at least one of the unlocked first position and the locked second position. In some embodiments, the biasing mechanism is configured to bias the movable locking element in both the unlocked first position and the locked second position and away from any position between the unlocked first position and the locked second position.

For example, the biasing mechanism may be a magnetic mechanism including at least one magnet. The magnetic mechanism may include at least one magnetic or ferromagnetic element mounted in the base member 122 and at least one magnetic or ferromagnetic element mounted in the movable locking element 150.

Thus, one or more recesses may be formed in the base member 122, which are configured to receive magnetic or ferromagnetic elements therein, and a cap or plug 146 may be used to cover the recesses and retain the magnetic or ferromagnetic elements therein. In the embodiment shown in the figures, the one or more recesses are formed into the base member 122 from the bottom surface thereof, and the plug 146 is fastened in a complementary shaped plug recess 147 in the bottom surface of the base member 122, as shown in FIGS. 6 and 7.

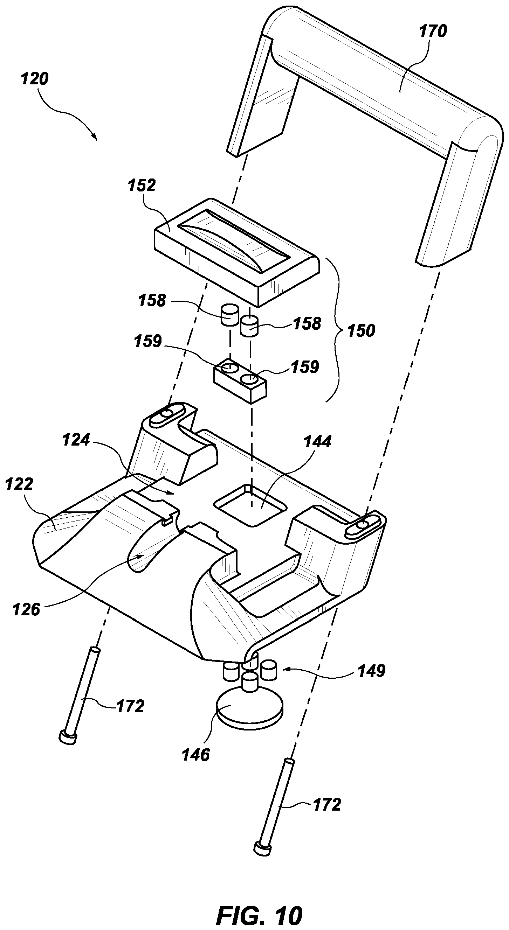

FIG. 10 is an exploded view of the tubing retention device 120. As shown therein, the protrusion 154 of the movable locking element 150 consists of a separate element from the main body 152 of the movable locking element 150. Two cylindrical recesses 159 are formed in a top surface of the protrusion 154, and a complementary sized and shaped cylindrical magnet 158 is positioned in each recess 159 in the protrusion 154. The protrusion 154 with the magnets 158 therein is then fastened to the lower surface of the main body 152 of the movable locking element 150 using, for example, any of a press fit, welding, brazing, an adhesive, fasteners, *etc.*

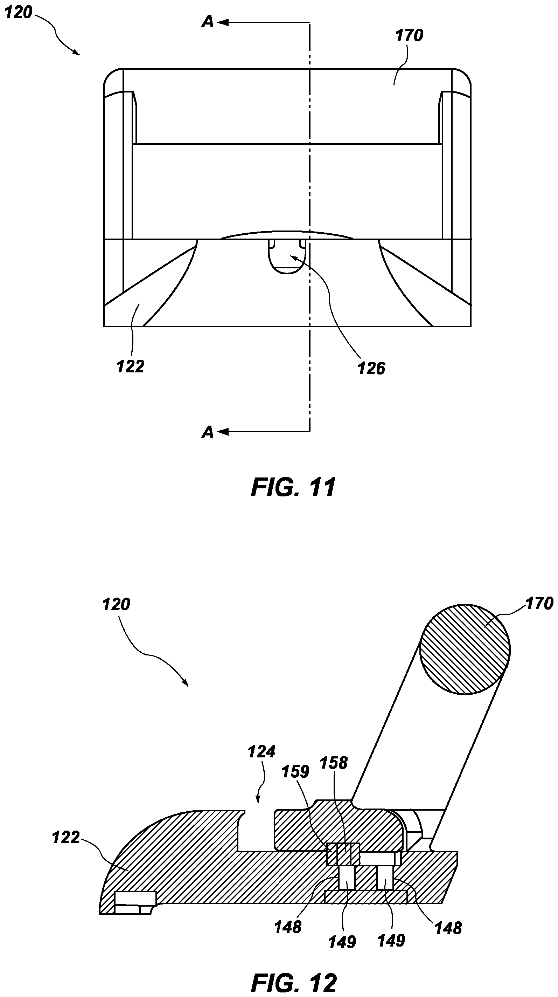

Four cylindrical recesses 148 (not visible in FIG. 10; see FIG. 12) are also formed in the bottom surface of the base member 122, and a complementary sized and shaped cylindrical magnet 149 is positioned in each recess 148 in the base member 122. The plug 146 is then fastened in the plug recess 147 in the bottom of the base member 122 to secure the magnets 149 in the corresponding magnet recesses 148.

FIG. 11 is a front view of the tubing retention device 120, and FIG. 12 is a cross-sectional side view of the tubing retention device 120 with the cross-section taken along the section line A-A in FIG. 11, which is located just to the right side of the second tubing recess 126. The relative positions of two of the magnets 149 in the magnet recesses 148 in the base member 122 are visible in FIG. 12, as is one of the magnets 158 in the magnet recess 159 in the protrusion 154 of the movable locking element 150. The movable locking element 150 is shown in the second locked position relative to the base member 122 in FIG. 12, at which location the magnet 158 in the protrusion 154 is vertically aligned with and attracted to the magnet 149 in the base member 122 closest to the first tubing recess 124. The movable locking element 150 can be manually moved by sliding the movable locking element away from the first tubing recess 124 by a user (to the right from the perspective of FIG. 12), against the magnetic biasing force, until the magnet 158 in the protrusion 154 is vertically aligned with and attracted to the magnet 149 in the base member 122 farthest from the first tubing recess 124 to place the movable locking element 150 in the first unlocked position.

In the embodiment shown in the figures, two sets of magnets, each set including one magnet 158 in the movable locking element 150 and two magnets 149 at different locations in the base member 122, work in the same identical manner in unison to provide increased magnetic bias or attraction to each of the first unlocked position and the second locked position of the movable locking element 150. One set of cooperating magnets is positioned along section line A-A in FIG. 11, as shown in FIG. 12. The second set of cooperating magnets is positioned along another plane on the opposite side of the second tubing recess 126 at an opposing symmetrical location as the section line A-A of FIG. 11, and a cross-sectional side view along that plane would be identical to the cross-sectional view of FIG. 12. In additional embodiments, the tubing retention device 120 may include only one such set of cooperating magnets, or three or more such sets of cooperating magnets.

Thus, the movable locking element 150 is magnetically biased toward both the unlocked first position and the locked second position by the cooperating magnets 149, 158, and is magnetically biased away from any position between the unlocked first position and the locked second position by the cooperating magnets 149, 158. Furthermore, any other configuration of cooperating magnetic or ferromagnetic elements may be employed in yet further embodiments of the present disclosure.

Returning briefly to the exploded view of FIG. 10, the optional handle 170 may be formed separately from the base member 122, and may be secured to the base member 122 using screws 172 that pass through the base member 122 into complementarily threaded recesses in the handle 170. Of course, any other method of attaching the optional handle 170 to the base member 122 may be employed, or the handle 170 may be integrally formed with the base member 122.

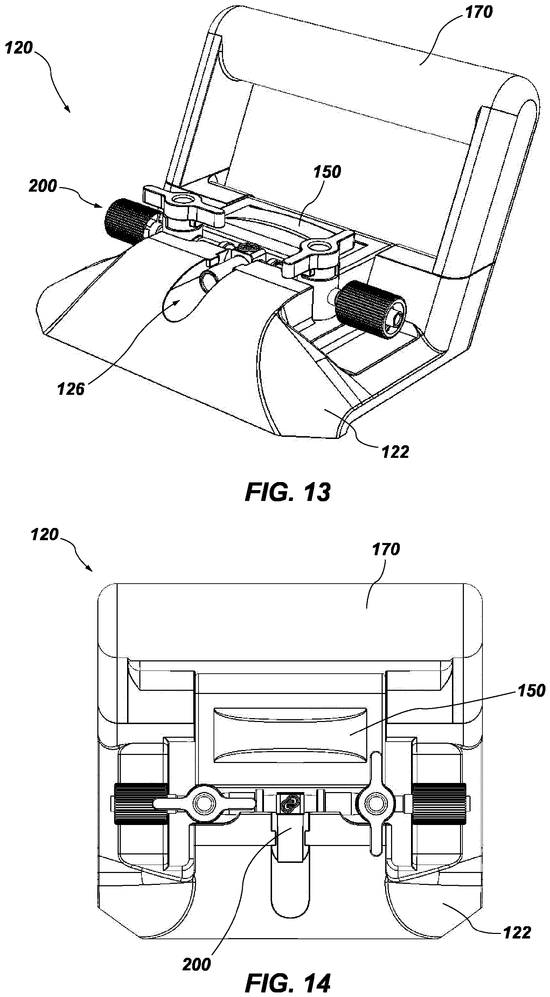

FIGS. 13 and 14 illustrate the T-shaped tubing assembly 200 of FIG. 15 secured within the tubing retention device 120 of FIGS. 1-12.

To secure the T-shaped tubing assembly within the tubing retention device 120, the movable locking element 150 is first moved to the first unlocked position. The T-shaped tubing assembly 200 is then positioned within the first tubing recess 124 and the second tubing recess 126 in the base member 122. In particular, the vertical branch 208 of the T-shaped manifold 206 is positioned in the second tubing recess 126, and the two horizontal branches 210 of the T-shaped manifold 206 are positioned in the first tubing recess 124.

As can be seen in FIG. 15, in some embodiments, the vertical branch 208 of the T-shaped manifold 206 may have lateral protrusions 209 such that the maximum width of the vertical branch 208 is wider than the spacing between the two second tubing retention protrusions 142 located over the second tubing recess 126. Thus, the vertical branch 208 of the T-shaped manifold 206 may be slid into the second tubing recess 126 from within the area of the first tubing recess 124, with the lateral protrusions 209 passing underneath the two second tubing retention protrusions 142 over the second tubing recess 126.

The movable locking element 150 carried by the base member 122 then may be moved from the unlocked first position to the locked second position as shown in FIGS. 13 and 14. Referring again to FIG. 15, the horizontal branches 210 of the T-shaped manifold 206 may also have protrusions 211 or enlarged regions that are configured to interfere with the first tubing retention protrusions 140 extending over the first tubing recess 124 when the T-shaped tubing assembly is positioned within the tubing retention device 120 and the movable locking element 150 is in the second locked position.

Thus, the movable locking element 150 locks the T-shaped tubing assembly 200 in the tubing retention device 120 while the movable locking element 150 is in the locked second position, as shown in FIGS. 13 and 14.

In this position, bottom surfaces of the valves 214 of the T-shaped tubing assembly 200 rest on and in contact with the bottom surface 134 within the first tubing recess 124, while the first relief shelf 128 and the second relief shelf 130 provide clearance for the luer connectors 212.

Either of the valves 214 of the T-shaped tubing assembly 200 may be opened or closed while manually pressing the valve against the bottom surface 134 of the first tubing recess 124.

Non-limiting example embodiments of the present disclosure may include:
Embodiment 1: A tubing retention device for retaining a T-shaped tubing assembly, the tubing retention device comprising: a base member having external surfaces defining a first tubing recess, a second tubing recess oriented at least substantially perpendicular to the first tubing recess, a first relief shelf disposed at a first end of the first tubing recess and recessed relative to a bottom surface of the first tubing recess, and a second relief shelf disposed at a second end of the first tubing recess and recessed relative to the bottom surface of the first tubing recess; and a movable locking element configured to move between an unlocked first position and a locked second position, the movable locking element configured to lock a T-shaped tubing assembly in the tubing retention device when the T-shaped tubing assembly is positioned within the first tubing recess and the second tubing recess and the movable locking element is moved to the locked second position.
Embodiment 2: The tubing retention device of Embodiment 1, wherein surfaces of the base member defining the first relief shelf and the second relief shelf are recessed relative to a surface of the base member defining a bottom surface within the first tubing recess by a distance in a range extending from 6.5 mm to 12.5 mm.
Embodiment 3: The tubing retention device of Embodiment 2, wherein the distance is about 9.5 mm.
Embodiment 4: The tubing retention device of any one of Embodiments 1-3, wherein a surface of the base member defining a bottom surface within the first tubing recess is planar.
Embodiment 5: The tubing retention device of any one of Embodiments 1-4, wherein surfaces of the base member defining the first relief shelf and the second relief shelf are planar.
Embodiment 6: The tubing retention device of any one of Embodiments 1-5, wherein a surface of the base member defining the second tubing recess is semicylindrical at a bottom of the second tubing recess and planar at opposing sides of the second tubing recess.
Embodiment 7: The tubing retention device of any one of Embodiments 1-6, wherein the base member further comprises at least one first tubing retention protrusion protruding over at least a portion of the first tubing recess, and at least one second tubing retention protrusion protruding over at least a portion of the second tubing recess, the first and second retention protrusions configured to hinder removal of a T-shaped tubing assembly from within the first tubing recess and the second tubing recess while the movable locking element is in the locked second position.
Embodiment 8: The tubing retention device of any one of Embodiments 1-7, further comprising a biasing mechanism configured to bias the movable locking element in at least one of the unlocked first position and the locked second position.
Embodiment 9: The tubing retention device of Embodiment 8, wherein the biasing mechanism is configured to bias the movable locking element in both the unlocked first position and the locked second position and away from any position between the unlocked first position and the locked second position.
Embodiment 10: The tubing retention device of any one of Embodiments 8-9, wherein the biasing mechanism comprises a magnetic mechanism including at least one magnet.
Embodiment 11: The tubing retention device of Embodiment 10, wherein the magnetic mechanism comprises at least one magnetic or ferromagnetic element mounted in the base member and at least one magnetic or ferromagnetic element mounted in the movable locking element.
Embodiment 12: The tubing retention device of Embodiment 11, wherein the at least one magnetic or ferromagnetic element mounted in the base member comprises at least two magnetic or ferromagnetic elements mounted in the base member at different locations in the base member, and wherein the at least one magnetic or ferromagnetic element mounted in the movable locking element is disposed adjacent a first magnetic or ferromagnetic element mounted in the base member when the movable locking element is in the unlocked first position, and the at least one magnetic or ferromagnetic element mounted in the movable locking element is disposed adjacent a second magnetic or ferromagnetic element mounted in the base member when the movable locking element is in the locked second position.
Embodiment 13: The tubing retention device of any one of Embodiments 1-12, further comprising a handle extending from the tubing retention device.
Embodiment 14: A fluid pump, comprising: a housing; and a tubing retention device according to any one of Embodiments 1-13 mounted to the housing.
Embodiment 15: A method of securing a T-shaped tubing assembly within a retention device according to any one of Embodiments 1-13, the method comprising: positioning a T-shaped tubing assembly within a first tubing recess and a second tubing recess in a base member, the second tubing recess oriented at least substantially perpendicular to the first tubing recess, the base member having external surfaces defining a first relief shelf disposed at a first end of the first tubing recess and recessed relative to a bottom surface of the first tubing recess, and a second relief shelf disposed at a second end of the first tubing recess and recessed relative to the bottom surface of the first tubing recess; and moving a movable locking element carried by the base member from an unlocked first position to a locked second position, the movable locking element locking the T-shaped tubing assembly in the retention device while the movable locking element is in the locked second position.
Embodiment 16: The method of Embodiment 15, wherein the base member further comprises at least one first tubing retention protrusion protruding over at least a portion of the first tubing recess, and at least one second tubing retention protrusion protruding over at least a portion of the second tubing recess, and wherein positioning the T-shaped tubing assembly within the first tubing recess and the second tubing recess comprises positioning the T-shaped tubing assembly under the at least one first tubing retention protrusion and the at least one second tubing retention protrusion before moving the movable locking element from the unlocked first position to the locked second position.
Embodiment 17: The method of Embodiment 15 or Embodiment 16, wherein the T-shaped tubing assembly comprises a first fluid valve and a second fluid valve, and wherein positioning the T-shaped tubing assembly within a first tubing recess and the second tubing recess in the base member comprises resting lower surfaces of the first fluid valve and the second fluid valve on the bottom surface of the first tubing recess.
Embodiment 18: The method of Embodiment 17, further comprising opening or closing at least one of the first fluid valve or the second fluid valve while pressing the at least one of the first fluid valve or the second fluid valve against the bottom surface of the first tubing recess.

The embodiments of the disclosure described above and illustrated in the accompanying drawings do not limit the scope of the disclosure, which is encompassed by the scope of the appended claims and their legal equivalents. Any equivalent embodiments are within the scope of this disclosure. Indeed, various modifications of the disclosure, in addition to those shown and described herein, such as alternate useful combinations of the elements described, will become apparent to those skilled in the art from the description. Such modifications and embodiments also fall within the scope of the appended claims and equivalents.

## Claims

1. A tubing retention device for retaining a T-shaped tubing assembly, the tubing retention device comprising:
a base member having external surfaces defining a first tubing recess, a second tubing recess oriented at least substantially perpendicular to the first tubing recess, a first relief shelf disposed at a first end of the first tubing recess and recessed relative to a bottom surface of the first tubing recess, and a second relief shelf disposed at a second end of the first tubing recess and recessed relative to the bottom surface of the first tubing recess; and
a movable locking element configured to move between an unlocked first position and a locked second position, the movable locking element configured to lock a T-shaped tubing assembly in the tubing retention device when the T-shaped tubing assembly is positioned within the first tubing recess and the second tubing recess and the movable locking element is moved to the locked second position.

2. The tubing retention device of claim 1, wherein surfaces of the base member defining the first relief shelf and the second relief shelf are recessed relative to a surface of the base member defining a bottom surface within the first tubing recess by a distance in a range extending from 6.5 mm to 12.5 mm, wherein the distance is preferably about 9.5 mm.

3. The tubing retention device of any one of the preceding claims, wherein a surface of the base member defining a bottom surface within the first tubing recess is planar.

4. The tubing retention device of any one of the preceding claims, wherein surfaces of the base member defining the first relief shelf and the second relief shelf are planar.

5. The tubing retention device of any one of the preceding claims, wherein a surface of the base member defining the second tubing recess is semicylindrical at a bottom of the second tubing recess and planar at opposing sides of the second tubing recess.

6. The tubing retention device of any one of the preceding claims, wherein the base member further comprises at least one first tubing retention protrusion protruding over at least a portion of the first tubing recess, and at least one second tubing retention protrusion protruding over at least a portion of the second tubing recess, the first and second retention protrusions configured to hinder removal of a T-shaped tubing assembly from within the first tubing recess and the second tubing recess while the movable locking element is in the locked second position.

7. The tubing retention device of any one of the preceding claims, further comprising a biasing mechanism configured to bias the movable locking element in at least one of the unlocked first position and the locked second position.

8. The tubing retention device of claim 7, wherein the biasing mechanism is configured to bias the movable locking element in both the unlocked first position and the locked second position and away from any position between the unlocked first position and the locked second position.

9. The tubing retention device of claim 7 or claim 8, wherein the biasing mechanism comprises a magnetic mechanism including at least one magnet.

10. The tubing retention device of claim 9, wherein the magnetic mechanism comprises at least one magnetic or ferromagnetic element mounted in the base member and at least one magnetic or ferromagnetic element mounted in the movable locking element.

11. The tubing retention device of claim 10, wherein the at least one magnetic or ferromagnetic element mounted in the base member comprises at least two magnetic or ferromagnetic elements mounted in the base member at different locations in the base member, and wherein the at least one magnetic or ferromagnetic element mounted in the movable locking element is disposed adjacent a first magnetic or ferromagnetic element mounted in the base member when the movable locking element is in the unlocked first position, and the at least one magnetic or ferromagnetic element mounted in the movable locking element is disposed adjacent a second magnetic or ferromagnetic element mounted in the base member when the movable locking element is in the locked second position.

12. The tubing retention device of any one of the preceding claims, further comprising a handle extending from the tubing retention device.

13. A fluid pump, comprising:
a housing; and
a tubing retention device mounted to the housing, the tubing retention device being in accordance with any one of the preceding claims.

14. A method of securing a T-shaped tubing assembly within a retention device, the method comprising:
positioning a T-shaped tubing assembly within a first tubing recess and a second tubing recess in a tubing retention device for retaining the T-shaped tubing assembly according to any one of claims 1 to 13; and
moving the movable locking element from an unlocked first position to a locked second position, the movable locking element locking the T-shaped tubing assembly in the retention device while the movable locking element is in the locked second position.

15. The method of claim 14, wherein the base member further comprises at least one first tubing retention protrusion protruding over at least a portion of the first tubing recess, and at least one second tubing retention protrusion protruding over at least a portion of the second tubing recess, and wherein positioning the T-shaped tubing assembly within the first tubing recess and the second tubing recess comprises positioning the T-shaped tubing assembly under the at least one first tubing retention protrusion and the at least one second tubing retention protrusion before moving the movable locking element from the unlocked first position to the locked second position.

16. The method of claim 15, wherein the T-shaped tubing assembly comprises a first fluid valve and a second fluid valve, and wherein positioning the T-shaped tubing assembly within a first tubing recess and the second tubing recess in the base member comprises resting lower surfaces of the first fluid valve and the second fluid valve on the bottom surface of the first tubing recess.

17. The method of claim 16, further comprising opening or closing at least one of the first fluid valve or the second fluid valve while pressing the at least one of the first fluid valve or the second fluid valve against the bottom surface of the first tubing recess.
